Europäisches Patentamt

European Patent Office

Office européen des brevets

(11) Veröffentlichungsnummer: **0 255 856 B1**

(19)

(12) **EUROPÄISCHE PATENTSCHRIFT**

(45) Veröffentlichungstag der Patentschrift: 06.03.91

(51) Int. Cl.⁵: **G01N 21/35**

(21) Anmeldenummer: 87107339.1

(22) Anmeldetag: 20.05.87

(54) Verfahren und Einrichtung zur Messung der Aldehydkonzentration in Abgasen.

(30) Priorität: 07.06.86 DE 3619301

(43) Veröffentlichungstag der Anmeldung:
17.02.88 Patentblatt 88/07

(45) Bekanntmachung des Hinweises auf die
Patenterteilung:
06.03.91 Patentblatt 91/10

(84) Benannte Vertragsstaaten:
AT CH DE FR IT LI NL

(56) Entgegenhaltungen:

ANALYTICAL CHEMISTRY, Band 58, Nr. 1,
Januar 1986, Seiten 68-72; L.P. HAACK et al.:
"Comparison of fourier transform infrared
spectrometry and 2,4-dinitrophenylhydrazine
impinger techniques for the mearsurement
of formaldehyde in vehicle exhaust"

STAUB-REINHALTUNG DER LUFT, Band 45,
Nr. 9, September 1985, Düsseldorf, Deutsch-
land, Seiten 423-425; H. KÖNIG:
"Photometrische Aldehydbestimmung"

STAUB-REINHALTUNG DER LUFT, Band 46,
Nr. 11, November 1986, Düsseldorf, Deutsch-
land, Seiten 490-496; L. GRUPINSKI: "Messen

von Gasen und Dämpfen mit IR-
Spektrometrie und Langweg-Gasküvetten"

TECHNISCHES MESSEN ATM, Heft 4, 1977,
Seiten 133-137; H. KLINGENBERG: "Mess-
und Prüfverfahren für Automobilabgase -
Übersicht und Kritik"

OPTICAL & QUANTUM ELECTRONICS, Band
8, 1976, Seite 89-93

(73) Patentinhaber: Ruhrgas Aktiengesellschaft
Huttropstrasse 60 Postfach 10 32 52
W-4300 Essen 1(DE)

(72) Erfinder: Kaesler Heribert Dipl.Ing.
Wabenweg 2
W-4630 Bochum(DE)

(74) Vertreter: Zenz, Joachim Klaus, Dipl.-Ing. et al
ZENZ & HELBER Patentanwälte Am Ruhrstein 1
W-4300 Essen 1(DE)

**Beschreibung**

Die Erfindung bezieht sich auf ein Verfahren und eine Einrichtung zur Messung der Aldehydkonzentration in Abgasen durch Infrarotspektroskopie.

Es ist bekannt, daß Aldehyde, insbesondere Formaldehyd im Abgas verschiedener Verbrennungsprozesse auftreten. Formaldehyd ist ein besonders reaktives Molekül, das leicht oxidiert, hydratisiert oder polymerisiert. Es neigt zur Kombination mit Atmosphärenbestandteilen und trägt dabei zur Bildung photochemischen Smogs bei.

Es wurde schon verschiedentlich vorgeschlagen, die Aldehydkonzentration in Abgasen mit Hilfe von Infrarotspektrometern oder mit Hilfe der Fouriertransformation-Infrarot-Spektrometrie (FT-IR) zu bestimmen. Aus Analytical Chemistry, Band 58, Nr. 1, Januar 1986 ist ein FT-IR-Verfahren bekannt, bei dem man die Formaldehydkonzentration von (mit Luft) verdünntem Abgas unter 3-Sekunden-Aufnahmezyklen mißt. Dieses bekannte Verfahren ist zwar zur kontinuierlichen oder quasi-kontinuierlichen Messung von Aldehyden geeignet, bedingt aber zur verläßlichen Messung der Aldehydkonzentration einen erheblichen gerätetechnischen und betrieblichen Aufwand.

Der Erfindung liegt die Aufgabe zugrunde, ein Verfahren und eine Einrichtung der eingangs genannten Art anzugeben, die eine on-line-Aldehydkonzentrationsmessung in Abgasen hoher Genauigkeit bei relativ geringem baulichen und betrieblichen Aufwand ermöglichen.

Bei der Lösung dieser Aufgabe geht die Erfindung von der Erkenntnis aus, daß bei der Infrarotmessung von Aldehyden eine nicht-vernachlässigbare Querempfindlichkeit gegen Kohlenwasserstoffe auftritt. Wie zu sehen sein wird, schaltet die Erfindung den Einfluß von Kohlenwasserstoffen bei der Messung der Aldehydkonzentration durch Infrarotmeßgeräte weitgehend aus.

Verfahrensmäßig besteht die Lösung der o.g. Aufgabe erfindungsgemäß darin, daß an einer Abgasprobe eine gegebenenfalls durch Kohlenwasserstoff beeinflußte erste IR-Messung durchgeführt wird, daß aus dieser oder einer zweiten Abgasprobe Aldehyd extrahiert und danach eine den Kohlenwasserstoffeinfluß bestimmende zweite IR-Messung an der von Aldehyd im wesentlichen befreiten Abgasprobe durchgeführt wird und daß ein Differenzsignal aus beiden Messungen abgeleitet und als Meßgröße zur Bestimmung der Aldehydkonzentration im Abgas verwendet wird.

Durch die Erfindung wird der Einfluß der Kohlenwasserstoffe auf die Aldehydkonzentrationsmessung mittels Infrarot-Meßgeräten durch einfache Differenzmessung des aldehydhaltigen und aldehydfreien Abgases eliminiert. Beide Messungen können in demselben IR-Meßgerät nach derselben Meßmethode und unter Verwendung übereinstimmender Auswerteschritte durchgeführt werden. Da Aldehyde (Formaldehyd, Acetaldehyd) gut wasserlöslich sind, können sie mit Hilfe einer der Meßküvette vorgeschalteten Waschflasche leicht aus dem Probengas entfernt werden (durch Auswaschen mit Wasser werden ca. 99 % des Aldehyd-Gehalts entfernt).

Das Abgas wird vorzugsweise unverdünnt den Messungen unterworfen.

In bevorzugter Weiterbildung der Erfindung wird die Probennahmestrecke, einschließlich Sonde, Probenleitung und der wenigstens einen Meßküvette beheizt.

In einer Verfahrensweise, die den Einsatz eines herkömmlichen IR-Meßgeräts ermöglicht, ist vorgesehen, daß dem Abgasstrom Abgasproben im wesentlichen kontinuierlich entnommen werden und in abwechselnden Meßphasen direkt oder nach Extraktion von Aldehyd indirekt der IR-Messung in derselben Küvette unterworfen werden.

In alternativer Verfahrensweise, die sich zur kontinuierlichen Messung der Aldehydkonzentration eignet, ist vorgesehen, daß zwei Abgasprobenströme kontinuierlich durch zwei Meßküvetten geleitet werden, wobei aus dem einen Abgasprobenstrom vor der IR-Messung Aldehyd ausgewaschen wird. Vorzugsweise werden die beiden Meßküvetten dabei gleichzeitig mit Licht von einem IR-Strahler des IR-Meßgeräts bestrahlt. Die beiden Abgasprobenströme können aus einer Probenleitung abgezweigt und im wesentlichen parallel zueinander den zugehörigen Meßküvetten zugeleitet werden, so daß aldehydhaltige und aldehydfreie Abgasproben praktisch gleichzeitig gemessen werden. Andererseits ist aber eine Verfahrensweise möglich, bei der eine Abgasprobe über eine Probennahmenstrecke einer ersten Meßküvette zugeleitet, danach zur Extraktion von Aldehyd durch einen Wascher und schließlich durch die zweite Meßküvette geleitet wird.

Sowohl die Signalverarbeitung zur Entwicklung des Differenzsignals als auch die Umsetzung der Extinktionsdifferenz in gewünschte Einheiten (mg/m$^3$ Aldehyd) lassen sich automatisieren, wobei vorzugsweise eine mit Rechner und gegebenenfalls Speicher versehene Auswerteeinrichtung Verwendung findet.

Bei der bevorzugt verwendeten Aldehydmeßlinie kann eine leichte Querempfindlichkeit der IR-Messung gegen Wasser auftreten. Diese läßt sich vor allem bei automatischer Auswertung einfach durch rechnerische Korrektur oder meßtechnisch über eine Taupunktmessung korrigieren, wobei die Taupunktmeßwerte vorzugsweise der Auswerteeinrichtung zur Korrektur des Differenzsignals zugeführt werden.

Die Einrichtung zur Messung der Aldehydkonzentration in Abgasen zeichnet sich erfindungsgemäß dadurch aus, daß ein Wascher zum Auswaschen des Aldehyds aus einer Abgasprobe vorgesehen ist und daß ein Eingangsanschluß des Waschers mit der Probennahmenstrecke verbindbar und ein Ausgangsanschluß des Waschers mit einer Meßküvette verbunden ist.

Diese Meßeinrichtung läßt sich als tragbares Meßgerät in relativ kompakter Bauform realisieren.

Weiterbildungen der Erfindung sind in den Unteransprüchen gekennzeichnet.

Im folgenden wird die Erfindung anhand von in der Zeichnung schematisch dargestellten Ausführungsbeispielen näher erläutert. In der Zeichnung zeigen:

Fig. 1 eine Einrichtung zur quasi-kontinuierlichen Messung der Aldehyd-Konzentration im Abgas unter Verwendung eines IR-Meßgeräts, das herkömmliche Ausbildung haben kann;

Fig. 2 eine Meßeinrichtung zur kontinuierlichen und gleichzeitigen Messung von aldehydhaltigem und aldehydfreiem Abgas in einem IR-Meßgerät; und

Fig. 3 eine Aldehyd-Meßeinrichtung ähnlich derjenigen gemäß Fig. 2, wobei eine Abgasprobe nacheinander durch eine erste Meßküvette, einen Wascher und eine zweite Meßküvette geleitet wird.

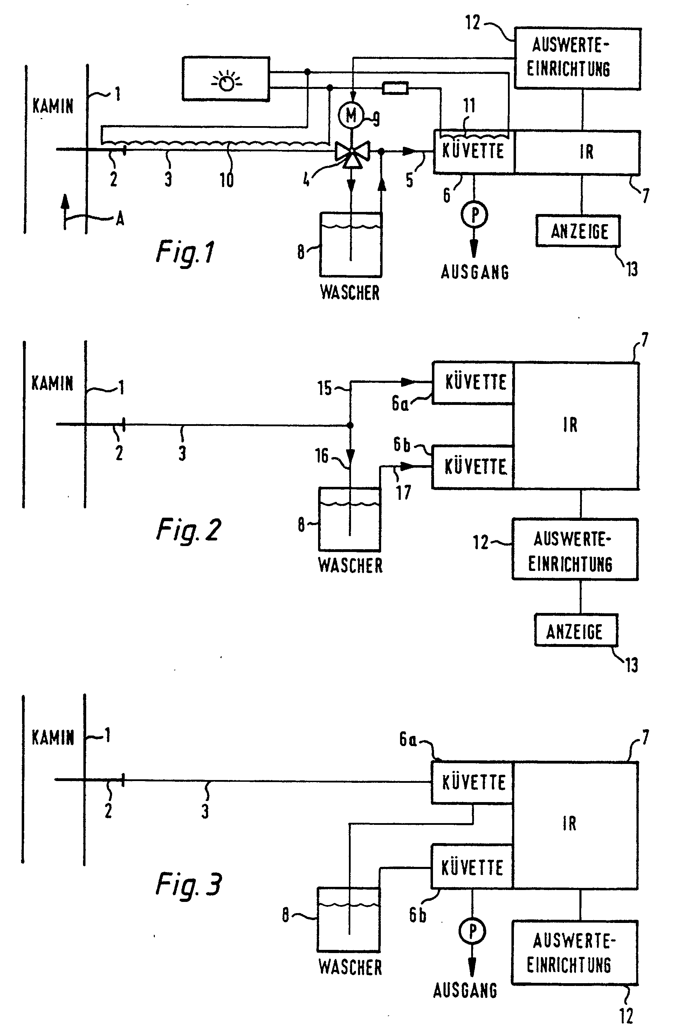

Einem in Fig. 1 mit einem Pfeil A angedeuteten Abgasstrom in einem Kamin 1 wird über eine Probennahmestrecke, bestehend aus einer Sonde 2 und einer Probenleitung 3, eine Abgasprobe entnommen. Die Abgasprobe wird über ein Dreiwegeventil 4 entweder über eine Leitung 5 direkt einer Meßküvette 6 eines IR-Meßgeräts 7 zugeführt oder indirekt über einen Wascher bzw. eine Waschflasche 8 und die Leitung 5 zur Meßküvette 6 geleitet. Die gesamte Probennahmestrecke von der Sonde 2 bis zur Küvette 6 ist über eine in Fig. 1 durch Heizspiralen 10, 11 dargestellte Heizvorrichtung beheizt, und zwar die Probenahmestrecke 2 ... 5 beispielsweise auf eine Temperatur 110° C und die Meßküvette auf eine Temperatur von 80° C.

Das Dreiwegeventil 4 kann von Hand umgeschaltet werden; in dem dargestellten Ausführungsbeispiel ist dem Dreiwegeventil ein Stellmotor 9 zugeordnet, dessen Schalttakt von einer zur Meßwertaufnahme mit dem IR-Gerät 7 verbundenen Auswerteeinrichtung 12 bestimmt wird. Bei Handbetätigung des Dreiwegeventils 4 genügt im Prinzip eine dem IR-Gerät nachgeschaltete Anzeigeeinrichtung 13 in Form eines Schreibers. Die Abgasprobe wird in bekannter Weise durch eine Pumpe P am Ausgang der Meßküvette 6 abgezogen.

Bei einem Versuchsaufbau wurde die Küvette 6 auf 80° C beheizt und hatte eine optische Weglänge von 21,25 m. Gemessen wurde bei einer Wellenlänge von 3,47 μm das Aldehydsignal, bei 3,82 μm die Nullinie und bei 7,66 μm das Wassersignal. Das Küvettenfenster bestand aus AgBr. Das Gasvolumen in der Küvette betrug 5,64 l. Der Gasdurchsatz in der Küvette lag zwischen 5 und 25 l/min.. Mit einem durch Flüssiginjektion hergestellten Prüfgas wurde das IR-Gerät auf Formaldehyd und ein Gemisch aus 90 % Formaldehyd und 10 % Acetaldehyd kalibriert. (Der Faktor zwischen beiden Kalibrierkurven lag bei 1,04.)

Die in Fig. 1 schematisch dargestellte Meßeinrichtung erlaubt eine von Einflüssen von Kohlenwasserstoff im Abgas unbeeinträchtigte Messung der Aldehydkonzentration, und zwar in einem quasi-kontinuierlichen Meßverfahren. Das Dreiwegeventil 4 wird dabei beispielsweise in einem Minutentakt derart umgeschaltet, daß der Küvette 6 abwechselnd das aldehydhaltige Abgas (über die Probennahmestrecke 2, 3 und die Leitung 5) und das durch den Wascher 8 geleitete und von Aldehyd weitgehend befreite Abgas zugeleitet wird. Das IR-Gerät mißt daher abwechselnd Aldehyd und Kohlenwasserstoffe und Kohlenwasserstoffe ohne Aldehyd. Das aus diesen beiden Meßsignalen beispielsweise in der Auswerteeinrichtung 12 entwickelte Differenzsignal wird in Extinktionseinheiten ausgegeben und mit Hilfe einer Kalibrierkurve in mg/m³ angegeben als Formaldehyd. Der Meßbereich liegt zwischen 0,5 und 200 mg/m³. Die Ausgabe kann auch direkt über einen dem IR-Gerät nachgeschalteten Schreiber 13 erfolgen. Bei automatischer Probenaufgabe und Auswertung mittels der Auswerteeinrichtung 12 kann das in der ersten Meßphase ermittelte erste Meßsignal für Aldehyd und Kohlenwasserstoffe gespeichert werden. Das Differenzsignal kann aus dem gespeicherten Wert und dem in der zweiten Meßphase ermittelten Kohlenwasserstoffwert durch einen geeigneten Komparator gebildet werden.

Eine alternative Meßanordnung, die eine kontinuierliche Messung der Aldehydkonzentration im Abgas gestattet, ist in Fig. 2 gezeigt. Die dem Kamin 1 über die Sonde 2 und die Probennahmeleitung 3 entnommene Abgasprobe wird über zwei Zweigleitungen 15 und 16 in praktisch parallelen Teilströmen einer ersten Meßküvette 6a und über einen Wascher 8 und eine Ausgangsleitung 17 einer zweiten Meßküvette 6b zugeleitet. Gegebenenfalls können den Leitungen 15 bzw. 16 separate Probennahmestrecken mit zwei Meßsonden zugeordnet sein, oder wenigstens eine der Leitungen 15 und 16 kann mit einem geeigneten einstellbaren Strömungswiderstand zur Einstellung der Abgas-Teilströme versehen sein.

Beide Meßküvetten 6a und 6b sind im IR-Gerät 7 so angeordnet, daß der zugehörige IR-Strahler sein Licht durch beide Küvetten gibt. Bei dieser Meßanordnung ist die Signalverarbeitung zur Gewinnung des gewünschten Differenzsignals besonders einfach, da beide Meßwertaufnahmen für das sowohl Aldehyd als auch Kohlenwasserstoff enthaltende Abgas und das Kohlenwasserstoff berücksichtigende Meßsignal gleichzeitig gewonnen werden.

Die Signalverarbeitung erfolgt über eine mit einem Rechner versehene Auswerteeinrichtung 12, der gegebenenfalls eine Anzeigeinheit 13 beispielsweise in Form eines Schreibers nachgeordnet ist.

Die in Fig. 3 schematisch dargestellte Meßanordnung entspricht derjenigen gemäß Fig. 2, mit der Ausnahme, daß die beiden Küvetten 6a und 6b nicht parallel, sondern unter Zwischenschaltung des Waschers 8 in Reihe geschaltet sind. Dadurch wird der gerätetechnische Aufwand für die kontinuierliche Messung der Aldehydkonzentration minimiert, da die Meßwertaufnahmen an jeweils den gleichen Abgasproben vorgenommen werden und die Durchflußmenge durch die Küvetten 6a und 6b über eine Pumpe P leicht eingestellt werden kann. Die jeweiligen Meßwertaufnahmen in den Küvetten 6a und 6b sind geringfügig phasenverschoben, was in der Praxis kaum ins Gewicht fällt und ohnehin über ein der Auswerteeinrichtung 12 zugeordnetes Rechnersystem berücksichtigt werden kann.

Bei den in den Figuren 2 und 3 dargestellten Meßanordnungen sind die Probennahmenstrecken bis hin zu den beiden Küvetten ähnlich beheizt wie bei der Meßeinrichtung gemäß Fig. 1. Alle in den Figuren 1 bis 3 dargestellten Meßeinrichtungen lassen sich als tragbares Gerät realisieren. Eine etwaige leichte Querempfindlichkeit von Wasserdampf kann über eine Taupunktmessung im Abgas korrigiert werden. Die Auswertung erfolgt in dem der Auswerteeinrichtung zugeordneten Rechner.

**Ansprüche**

1. Verfahren zur Messung der Aldehydkonzentration in Abgasen durch Infrarotspektroskopie, **dadurch gekennzeichnet,** daß an einer Abgasprobe eine gegebenenfalls durch Kohlenwasserstoffe beeinflußte erste IR-Messung durchgeführt wird, daß aus dieser oder einer zweiten Abgasprobe Aldehyd extrahiert und danach eine den Kohlenwasserstoffeinfluß bestimmende zweite IR-Messung an der von Aldehyd im wesentlichen befreiten Abgasprobe durchgeführt wird und daß ein Differenzsignal aus beiden Messungen abgeleitet und als Meßgröße zur Bestimmung der Aldehydkonzentration im Abgas verwendet wird.

2. Verfahren nach Anspruch 1, dadurch gekennzeichnet, daß das Aldehyd durch Auswaschen mit Wasser weitgehend extrahiert wird.

3. Verfahren nach Anspruch 1 oder 2, dadurch gekennzeichnet, daß das Abgas unverdünnt den Messungen unterworfen wird.

4. Verfahren nach einem der Ansprüche 1 bis 3, dadurch gekennzeichnet, daß die Probennahmestrecke (2 ... 5) vorzugsweise auf eine Temperatur zwischen 70 und 120° C beheizt wird.

5. Verfahren nach einem der Ansprüche 1 bis 4, dadurch gekennzeichnet, daß eine im wesentlichen kontinuierlich dem Abgasstrom entnommene Abgasprobe in abwechselnden Meßphasen entweder direkt oder nach Extraktion von Aldehyd indirekt der IR-Messung in derselben Küvette unterworfen wird.

6. Verfahren nach einem der Ansprüche 1 bis 5, dadurch gekennzeichnet, daß zwei Abgasprobenströme kontinuierlich durch zwei Meßküvetten geleitet werden, wobei aus dem einen Abgasprobenstrom vor der IR-Messung Aldehyd ausgewaschen wird.

7. Verfahren nach Anspruch 6, dadurch gekennzeichnet, daß die beiden Meßküvetten gleichzeitig mit Licht von einem IR-Strahler bestrahlt werden.

8. Verfahren nach Anspruch 6 oder 7, dadurch gekennzeichnet, daß die beiden Abgasprobenströme parallel zueinander den zugehörigen Meßküvetten zugeleitet werden.

9. Verfahren nach Anspruch 6 oder 7, dadurch gekennzeichnet, daß eine Abgasprobe zunächst durch eine erste Meßküvette, danach zur Extraktion von Aldehyd durch einen Wascher und schließlich durch eine zweite Meßküvette geleitet wird.

10. Verfahren nach einem der Ansprüche 1 bis 10, dadurch gekennzeichnet, daß aus den Meßwerten der aldehydhaltigen und aldehydfreien Abgasproben, gegebenenfalls nach Zwischenspeicherung, eine dem Differenzsignal proportionale Größe in einem Rechner berechnet und zur Bestimmung der Aldehydkonzentration verarbeitet wird.

11. Verfahren nach einem der Ansprüche 1 bis 10, gekennzeichnet durch eine zusätzliche Taupunktmessung im Abgas zur Korrektur einer Querempfindlichkeit der IR-Messung gegen Wassermoleküle.

12. Einrichtung zur Messung der Aldehydkonzentration in Abgasen, mit einer eine Abgassonde (2) enthaltenden Probennahmestrecke (2 ... 5; 15, 16) und einem Infrarotmeßgerät (7), dessen Meßküvette (6; 6a 6b) mit der Probennahmestrecke verbindbar ist, **dadurch gekennzeichnet, daß** ein Wascher (8) zum Auswaschen des Aldehydes aus einer Abgasprobe vorgesehen ist, daß ein Eingangsanschluß des Waschers mit der Probennahmestrecke verbindbar und ein Ausgangsanschluß des Waschers mit einer Meßküvette (6; 6b) des IR-Meßgeräts (7) verbunden ist.

13. Einrichtung nach Anspruch 12, dadurch gekennzeichnet, daß in einer Probenleitung (3, 5) der Probennahmestrecke ein Mehrwegeventil (4) angeordnet ist, dessen Durchlaßweg entweder direkt mit der Meßküvette (6) oder über den Wascher (8) indirekt mit der Meßküvette verbindbar ist.

14. Einrichtung nach Anspruch 13, dadurch gekennzeichnet, daß das Mehrwegeventil (14) durch einen von einem Umschalttaktgeber gesteuerten Stellmotor (9) betätigbar ist.

15. Einrichtung nach Anspruch 12, dadurch gekennzeichnet, daß zwei Meßküvetten (6a 6b), von denen einer (6b) ein Wascher (8) vorgeschaltet ist, im Strahlengang des IR-Strahlers eines IR-Meßgeräts (7) angeordnet sind.

16. Einrichtung nach Anspruch 15, dadurch gekennzeichnet, daß die beiden Meßküvetten (6a 6b) parallel zueinander in der Probennahmestrecke (3, 15, 16) angeordnet sind.

17. Einrichtung nach Anspruch 15, dadurch gekennzeichnet, daß die erste Meßküvette (6a), der Wascher (8) und die zweite Küvette (6b) in Reihe hintereinander angeordnet sind.

18. Einrichtung nach einem der Ansprüche 12 bis 17, dadurch gekennzeichnet, daß dem IR-Meßgerät (7) eine einen Rechner enthaltende Auswerteeinrichtung (12) zum Umsetzen der Meßsignale in eine für die Aldehydkonzentration im Abgas charakteristische Meßgröße zugeordnet ist.

19. Einrichtung nach den Ansprüchen 14 und 18, dadurch gekennzeichnet, daß die Auswerteeinrichtung (12) einen Umschalttaktgeber zur Steuerung des Stellmotors (9) des Mehrwegeventils (4) enthält.

20. Einrichtung nach einem der Ansprüche 12 bis 19, dadurch gekennzeichnet, daß eine Heizvorrichtung (10, 11) zum Beheizen der Probennahmestrecke (2 ... 5; 2, 3, 15, 16, 17) und der wenigstens einen Meßküvette (6; 6a, 6b) vorgesehen ist.

## Claims

1. A method of measuring the aldehyde concentration in waste gases by infrared spectroscopy characterized in that a waste gas sample is tested by a first infrared measurement which may be influenced by hydrocarbons, aldehyde is extracted from said or a second waste gas sample, a second infrared measurement determining the influence of the hydrocarbons is thereupon performed on the waste gas sample from which the aldehyde has substantially been removed, and a differential signal is derived from the two measurements and used as a variable for determining the aldehyde concentration in the waste gas.

2. A method according to claim 1 characterized in that the aldehyde is substantially extracted by washing using water.

3. A method according to claim 1 or 2 characterized in that the waste gas is left undiluted for measurement.

4. A method according to any one of claims 1 through 3 characterized in that the sampling section (2 ... 5) is heated preferably to a temperature of between 70 and 120° C.

5. A method according to any one of claims 1 through 4 characterized in that a waste gas sample taken substantially continuously from the waste gas stream is measured by infrared measurement in the same cell in cycles wherein direct measurements and indirect measurements following the extraction of aldehyde alternate.

6. A method according to any one of claims 1 through 5 characterized in that two streams of waste gas samples are passed continuously through two measuring cells, the aldehyde being washed out of one of said waste gas streams prior to the infrared measurement.

7. A method according to claim 6 characterized in that the two cells are simultaneously exposed to light from an infrared radiator.

8. A method according to claim 6 or 7 characterized in that the two waste gas sample streams are passed into their appropriate cells parallel to each other.

9. A method according to claim 6 or 7 characterized in that a waste gas sample is initially passed through a first cell, then through a washer for the aldehyde to be extracted, and finally through a second cell.

10. A method according to any one of claims 1 through 10 characterized in that a value proportionate to the difference between the value measured for the aldehyde-containing waste gas sample and the value measured for the aldehyde-free waste gas sample, said two values possibly being stored, is calculated by a computer determining the aldehyde concentration.

11. A method according to any one of claims 1 through 10 characterized in that the dew-point measurement of the waste gas is additionally measured to make a correction for any crosssensitivity of the infrared measuring device to water molecules.

12. An apparatus for measuring the aldehyde concentration in waste gases comprising a sampling section (2 ... 5; 15, 16) containing a waste gas probe (2) and an infrared measuring device (7) provided with a cell (6; 6a 6b) which may be coupted with the sampling section characterized in that a washer (8) is provided for washing the aldehyde out of a waste gas sample, a washer inlet fitting is provided and may be coupled with the sampling section and a fitting at the outlet of the washer is coupled with a cell (6; 6b) of the infrared measuring device (7).

13. An apparatus according to claim 12 characterized in that a multiway valve (4) is arranged in a sample line (3, 5) of the sampling section, the passage therethrough allowing coupling either directly with the cell (6) or indirectly with the cell across the washer (8).

14. An apparatus according to claim 13 characterized in that the multiway valve (4) is actuated by an actuator (9) controlled by a change-over cycling device.

15. An apparatus according to claim 12 characterized in that two cells (6a 6b), one of said two cells (6b) being arranged upstream of a washer (8), are arranged in the beam path of the infrared radiator of an infrared measuring device (7).

16. An apparatus according to claim 15 characterized in that the two cells (6a 6b) are arranged parallel to each other in the sampling section (3, 15, 16).

17. An apparatus according to claim 15 characterized in that the first cell (6a), the washer (8) and the second cell (6b) are arranged in series.

18. An apparatus according to any one of claims 12 through 17 characterized in that the infrared measuring device (7) is provided with a processing device (12) comprising a computer for converting the signals representing the measured variables into values which are characteristic of the aldehyde concentration in the waste gas.

19. An apparatus according to any one of claims 14 through 18 characterized in that the processing device (12) is provided with a changeover cycling device for controlling the actuator (9) of the multiway valve (4).

20. An apparatus according to any one of claims 12 through 19 characterized in that a heating device (10, 11) is provided for heating the sampling section (2 ... 5; 2, 3, 15, 16, 17) and the at least one cell (6; 6a, 6b).

**Revendications**

1. Procédé pour mesurer la concentration d'aldéhyde dans des fumées par spectroscopie infrarouge, caractérisé par le fait qu'un échantillon de fumées est soumis à une spectroscopie infrarouge, influencée, le cas échéant, par des hydrocarbures, que de l'aldéhyde est extrait dudit échantillon ou d'un deuxième échantillon de fumées et que, ensuite, l'échantillon de fumées substantiellemet exempt d'aldéhydes est soumis à une deuxième spectrospie infrarouge déterminant l'influence des hydrocarbures, et qu'un signal de différence entre les deux mesures est déduit et utilisé comme grandeur pour déterminer la concentration d'aldéhyde dans les fumées.

2. Procédé selon la revendication 1, caractérisé par le fait que l'aldéhyde est substantiellement extrait des fumées par lavage à l'eau.

3. Procédé selon l'une quelconque des revendications 1 ou 2, caractérisé par le fait que les fumées sont soumises à des mesures à l'état non dilué.

4. Procédé selon l'une quelconque des revendications 1 à 3, caractérisé par le fait que le tronçon de prise d'échantillons (2 ... 5) est chauffé de préférence à une température entre 70 et 120° C.

5. Procédé selon l'une quelconque des revendications 1 à 4, caractérisé par le fait qu'un échantillon extrait en principe continuellement du flux de fumées est soumis, à des cyles de mesures alternants, à une spectroscopie infrarouge dans la même cuvette soit directement, soit indirectement après l'extraction d'aldéhydes.

6. Procédé selon l'une quelconque des revendications 1 à 5, caractérisé par le fait que deux flux d'échantillon de fumées passent continuellement par deux cuvettes, l'aldéhyde d'un des deux flux d'échantillon de fumées étant extrait avant la spectroscopie infrarouge.

7. Procédé selon la revendication 6, caractérisé par le fait que les deux cuvettes sont exposées en même temps aux rayons d'une seule source infrarouge.

8. Procédé selon l'une quelconque des revendications 6 ou 7, caractérisé par le fait que les deux flux d'échantillon de fumées sont acheminées en parallèle aux cuvettes correspondantes.

9. Procédé selon l'une quelconque des revendications 6 et 7, caractérisé par le fait qu'un échantillon de fumées passe d'abord à travers d'une première cuvette, ensuite à travers d'un laveur pour l'extraction d'aldéhydes et enfin à travers d'une deuxième cuvette.

10. Procédé selon l'une quelconque des revendications 1 à 9, caractérisé par le fait qu'à partir des valeurs de mesure de l'échantillon de fumées contenant de l'aldéhyde et de l'échantillon de fumée exempt d'aldéhyde, après, le cas échéant, mis en mémoire intermédiaire, une valeur proportionelle au signal de différence est calculée dans un calculateur est traitée pour la détermination de la concentration d'aldéhyde.

11. Procédé selon l'une quelconque des revendications 1 à 10, caractérisé par une mesure additionelle du point de rosée dans les fumées pour corriger la sensibilité de la spectroscopie infrarouge vis-à-vis des molécules d'eau.

12. Dispositif pour mesurer la concentration d'aldéhyde dans des fumées comprenant un tronçon de prise d'échantillon (2 ... 5, 15, 16) muni d'une sonde de fumées (2), et un spectromètre infrarouge (7) dont la cuvette (6, 6a, 6b) peut être raccordée au tronçon de prise d'échantillon, caractérisé par le fait qu'un laveur (8) est prévu pour l'extraction d'aldéhydes de l'échantillon de fumées, que l'entrée du laveur peut être raccordée au tronçon de prise d'échantillon et que la sortie du laveur peut être raccordée à une cuvette (6, 6b) du spectromètre (7).

13. Dispositif selon la revendication 12, caractérisé par le fait qu'un robinet à plusieurs voies (4) est disposé dans une conduite d'échantillon (3, 5) du tronçon de prise d'échantillon, laissant passer le flux soit directement, soit indirectement à travers du laveur (8) à la cuvette (6).

14. Dispositif selon la revendication 13, caractérisé par le fait que le robinet à plusieurs voies (4) est actionné par un moteur (9) commandé par un générateur d'impulsions.

15. Dispositif selon la revendication 12, caractérisé par le fait que deux cuvettes (6a, 6b), dont l'une (6b) étant disposée en aval d'un laveur (8), sont disposées dans le passage des rayons de la source infrarouge du spectromètre infrarouge (7).

16. Dispositif selon la revendication 15, caractérisé par le fait que les deux cuvettes (6a, 6b) sont disposées en parallèle dans le tronçon de prise d'échantillon (3, 15, 16).

17. Dispositif selon la revendication 15, caractérisé par le fait que la première cuvette (6a), le laveur (8) et la deuxième cuvette (6b) sont disposés en série les uns derrière les autres.

18. Dispositif selon l'une quelconque des revendications 12 à 17, caractérisé par le fait qu'au spectromètre infrarouge (7) est attribué un dispositif de dépouillement (12) comprenant un calculateur pour transformer les signaux de mesure en grandeur caractéristique pour la concentration d'aldéhyde dans les fumées.

19. Dispositif selon l'une quelconque des revendications 14 à 18, caractérisé par le fait que le dispositif de dépouillement (12) comprend un générateur d'impulsions pour actionner le moteur (9) du robinet à plusieurs voies (4).

20. Dispositif selon l'une quelconque des revendications 12 à 19, caractérisé par le fait qu'un chauffage (10, 11) est prévu pour chauffer le tronçon de prise d'échantillon (2 ... 5, 2, 3, 15, 16, 17) et au moins une cuvette (6, 6a, 6b).

Fig.1

Fig.2

Fig.3